# FASCICULE DE BREVET EUROPEEN

(11) **EP 3 299 055 B1**
(45) Date de publication et mention de la délivrance du brevet: **18.12.2019**
(21) Numéro de dépôt: 17305864.5
(22) Date de dépôt: 04.07.2017
(51) Int. Cl.: A61M 16/00, A61H 31/00, A61M 16/20

(54) **APPAREIL D'ASSISTANCE RESPIRATOIRE AVEC DÉTECTION AUTOMATIQUE DU MODE DE MASSAGE CARDIAQUE MANUEL OU AUTOMATIQUE**
GERÄT ZUR ATMUNGSUNTERSTÜTZUNG MIT AUTOMATISCHER ERFASSUNG DES MANUELLEN ODER AUTOMATISCHEN HERZMASSAGEMODUS
BREATHING ASSISTANCE APPARATUS WITH AUTOMATIC DETECTION OF MANUAL OR AUTOMATIC CARDIAC MASSAGE MODE

(30) Priorité: 22.09.2016 FR 1658889
(43) Date de publication de la demande: 28.03.2018
(73) Titulaire: Air Liquide Medical Systems, 92160 Antony (FR)
(72) Inventeur: DERMEL, Marius, 75003 PARIS (FR); JACQUOT, Eric, 92160 ANTONY (FR); RICHARD, Jean-Christophe, 92160 ANTONY (FR); RIGOLLOT, Marceau, 92120 MONTROUGE (FR)
(74) Mandataire: Pittis, Olivier

(56) Documents cités:
- EP-A1- 0 839 545
- FR-A1- 2 802 431
- FR-A1- 3 000 893

## Description

L'invention concerne un appareil de ventilation artificielle conçu pour être utilisé lors d'un massage cardiaque sur un patient ventilé, capable de déterminer automatiquement le mode de réalisation du massage cardiaque pratiqué sur un patient, c'est-à-dire manuel ou massage automatique, et de délivrer une ventilation adaptée au massage détecté.

Il est usuel d'utiliser un appareil de ventilation artificielle, encore appelé appareil d'assistance respiratoire ou ventilatoire, ou plus simplement ventilateur médical, pour apporter une assistance respiratoire, c'est-à-dire une ventilation artificielle, à une personne ayant des difficultés ou une incapacité à respirer seule, notamment une personne en arrêt cardio-pulmonaire.

En effet, au cours d'un massage cardiaque réalisé sur une personne, i.e. un patient, en arrêt cardio-pulmonaire, les compressions de la cage thoracique par un secouriste, médecin, pompier ou analogue génèrent une ventilation pulmonaire substantielle mais généralement insuffisante. Elle doit dès lors être complétée par une ventilation mécanique. Un appareil utilisable à cette fin est décrit par le document FR-A-3000893. Le document FR2802431, lui, décrit un appareil d'assistance respiratoire détectant les paramètres de l'assistance manuelle pour les reproduire au moins partiellement durant l'assistance automatique.

Quand les compressions thoraciques sont interrompues, après reprise d'activité circulatoire spontanée du patient ou après un choc électrique par exemple, la ventilation du patient diminue brutalement, alors que ses besoins en oxygène augmentent. Un complément de ventilation est alors aussi nécessaire et doit être apporté par ventilation mécanique.

Les poumons et le cœur se trouvent tous les deux dans la cage thoracique. La ventilation mécanique, par la mise en place d'une pression expiratoire positive ou PEP, permet de protéger les poumons en y maintenant un volume minimal d'air, la PEP générée par le ventilateur permettant de limiter la dissipation par les voies aériennes de l'énergie du massage.

Les cycles inspiratoires, de préférence à pression contrôlée, générés par la ventilation mécanique, provoquent une augmentation de la pression au sein des poumons. Cette augmentation de la pression engendre une remontée de la cage thoracique et la présence au sein des poumons d'un volume supérieur au volume en position de repos appelé « capacité résiduelle fonctionnelle ».

D'une façon générale, une ventilation mécanique génère des cycles inspiratoires de façon répétée, pendant le massage cardiaque, empêchant ainsi un massage se déroulant systématiquement en dessous de la capacité résiduelle fonctionnelle.

Le massage cardiaque peut donc être réalisé :
- soit manuellement par un praticien et il est alors dit « manuel » ;
- soit avec un dispositif de compression thoracique automatisé, telle une planche à masser, et il est alors dit « automatique ».

Les dispositifs de compressions thoraciques automatisées, couramment appelés « planches à masser automatiques » ou « dispositifs de compressions thoraciques mécanisés », délivrent un massage régulier. Certains utilisent un piston ou une sangle.

La fréquence et la profondeur des compressions sont constantes, par exemple la fréquence est souvent de 100 compressions par minute et la profondeur de 5 centimètres.

Ces planches délivrent donc le même massage quelle que soit la morphologie du patient pris en charge. Il s'ensuit que le massage délivré est trop violent pour de nombreux patients et peut provoquer chez eux des lésions pulmonaires résultant de volumes pulmonaires en dessous de la capacité résiduelle fonctionnelle.

Il est donc nécessaire de pouvoir connaître le type de massage pratiqué sur un patient de manière à pouvoir rétroagir sur l'appareil de ventilation artificielle et assurer ainsi une ventilation adaptée prévenant les lésions pulmonaires chez le patient. Idéalement, cette reconnaissance ou détermination du type de massage, i.e. manuel ou automatique, par exemple au moyen d'une planche à masser, doit être opérée automatiquement par l'appareil de ventilation artificielle lui-même.

Le problème qui se pose est dès lors de proposer un appareil de ventilation artificielle, c'est-à-dire un appareil d'assistance respiratoire ou ventilateur médical, permettant de détecter le mode de réalisation des compressions thoraciques, c'est-à-dire mode manuel ou mode automatique, et de délivrer une ventilation, efficace et protectrice, adaptée au type de massage.

La solution de l'invention est un appareil d'assistance respiratoire, c'est-à-dire un ventilateur médical, comprenant un circuit de gaz conçu pour acheminer un flux de gaz, en particulier un flux d'air, comprenant :
- des moyens de mesure agencés pour mesurer au moins un paramètre de flux représentatif du flux de gaz acheminé par le circuit de gaz et configurés pour convertir ledit au moins un paramètre de flux en au moins un signal de flux représentatif du flux de gaz,
- des moyens de traitement de signal aptes à et conçus pour traiter ledit au moins un signal de flux fourni par les moyens de mesure, et pour en déduire une information représentative d'un mode de massage cardiaque manuel ou d'un mode de massage cardiaque automatique,
- des moyens de mémorisation configurés pour mémoriser plusieurs modes ventilatoires comprenant:
   i) un premier mode ventilatoire, encore appelé « mode pour massage manuel » comprenant des premiers paramètres de ventilation applicables en cas de massage cardiaque manuel, et
   ii) un second mode ventilatoire encore appelé « mode pour massage automatique » comprenant des seconds paramètres de ventilation applicables en cas de massage cardiaque automatique, et
- des moyens de sélection de mode ventilatoire aptes à et conçus pour, i.e. configurés pour, retrouver et sélectionner au sein des moyens de mémorisation, le premier ou le deuxième mode ventilatoire mémorisé, en fonction de l'information représentative d'un mode de massage cardiaque manuel ou automatique fournie par les moyens de traitement de signal.

Dans le cadre de la présente invention, on appelle « mode ventilatoire », un ensemble de paramètres ventilatoires, tel que pression, débit, volume de gaz délivré... , utilisés pour configurer l'appareil de ventilation et déterminer/fixer ainsi les caractéristiques de la ventilation délivrée au patient. A ces paramètres ventilatoires peuvent s'ajouter des valeurs de monitorage, tel que le volume inspiré, le volume expiré, les pressions, la concentration en CO2, la concentration en oxygène, des échanges gazeux entre le patient et l'appareil de ventilation...

Selon le cas, l'appareil médical de l'invention peut comprendre l'une ou plusieurs des caractéristiques techniques suivantes :
- les moyens de mesure du paramètre de flux comprennent au moins un capteur de pression de gaz ou un capteur de débit de gaz.
- les moyens de mesure du paramètre de flux opèrent une mesure d'au moins un paramètre de flux représentatif du flux de gaz au sein d'un conduit d'acheminement de gaz du circuit de gaz.
- les moyens de mesure du paramètre de flux opèrent une mesure d'au moins un paramètre de flux choisi parmi la pression et le débit du gaz, de préférence la pression de gaz.
- les moyens de mémorisation configurés pour mémoriser un premier mode ventilatoire, encore appelé « mode pour massage manuel », comprenant des premiers paramètres de ventilation comprenant des premières valeurs de pression.
- les moyens de mémorisation configurés pour mémoriser un second mode ventilatoire, encore appelé « mode pour massage automatique », comprenant des seconds paramètres de ventilation comprenant des secondes valeurs de pression.
- les moyens de mémorisation configurés pour mémoriser un premier mode ventilatoire comprenant des premiers paramètres de ventilation comprenant des valeurs de première pression basse (PB1) et de première pression haute (PH1), avec PH1>PB1.
- les moyens de mémorisation configurés pour mémoriser un second mode ventilatoire comprenant des valeurs de deuxième pression basse (PB2) et de deuxième pression haute (PH2), avec PH2≥PH1>PB2 et PB2>PB1.
- il comprend des moyens d'alimentation en gaz pour délivrer un flux de gaz dans le circuit de gaz, c'est-à-dire que les moyens d'alimentation en gaz sont conçus pour ou configurés pour fournir un flux de gaz au circuit de gaz.
- il comprend des moyens de pilotage commandant les moyens d'alimentation en gaz en fonction du mode ventilatoire sélectionné par les moyens de sélection de mode ventilatoire.
- les moyens de pilotage comprennent une première carte électronique.
- les moyens de pilotage comprennent au moins un premier microprocesseur.
- les moyens de pilotage comprennent au moins un premier (micro)contrôleur, de préférence au moins un premier (micro)contrôleur mettant en œuvre au moins un premier algorithme.
- les moyens de traitement de signal comprennent une deuxième carte électronique.
- les moyens de traitement de signal comprennent au moins un deuxième microprocesseur.
- les moyens de traitement de signal comprennent au moins un deuxième (micro)contrôleur, de préférence au moins un deuxième (micro)contrôleur mettant en œuvre au moins un deuxième algorithme.
- la première et la deuxième carte électronique forment une seule et même carte électronique, c'est-à-dire une carte électronique unique.
- les moyens de pilotage commandent les moyens d'alimentation en gaz pour appliquer le premier mode ventilatoire comprenant des premiers paramètres de ventilation, en particulier les valeurs de première pression basse (PB1) et de première pression haute (PH1), avec PH1>PB1, en cas de massage cardiaque manuel détecté.
- les moyens de pilotage commandent les moyens d'alimentation en gaz pour appliquer le second mode ventilatoire comprenant des seconds paramètres de ventilation, en particulier les valeurs de deuxième pression basse (PB2) et de deuxième pression haute (PH2), avec PH2≥PH1>PB2 et PB2>PB1, en cas de massage cardiaque automatique détecté.
- les moyens d'alimentation en gaz comprennent une micro-soufflante motorisée équipée ou non d'une vanne inspiratoire ou une canalisation d'alimentation en gaz équipée d'une vanne inspiratoire, de préférence une micro-soufflante équipée d'un moteur électrique.
- il comprend des moyens d'affichage conçus pour afficher ladite au moins une information représentative du mode de massage cardiaque manuel ou automatique fournie par les moyens de traitement de signal, c'est-à-dire l'information relative au mode de délivrance du massage cardiaque appliqué au patient et provenant des moyens de traitement de signal.
- les moyens d'affichage comprennent au moins un afficheur numérique.
- les moyens de traitement de signal sont configurés pour déterminer l'amplitude (A) des variations de pression ou de débit engendrées par un massage cardiaque en déterminant la différence entre la pression maximale (Pmax) ou le débit maximal (Qmax), respectivement, et la pression minimale (Pmin) ou le débit minimal (Qmin) atteints, lors des phases de compression et de relâchement de la cage thoracique du patient opérées pendant le massage cardiaque.
- les moyens de traitement de signal sont en outre configurés pour :
   i) déterminer une information de régularité de massage (R1) représentative de la régularité du massage cardiaque en déterminant la variabilité de l'amplitude (A) pendant la ventilation et le massage cardiaque,
   ii) comparer cette information de régularité de massage (R1) à un seuil mémorisé (S1), et
   iii) déduire que le massage cardiaque est :
      - réalisé manuellement lorsque l'information de régularité de massage (R1) est supérieure à ce seuil (S1), i.e. R1>S1, ou
      - réalisé automatiquement, c'est-à-dire par une planche à masser ou analogue, lorsque l'information de régularité de massage (R1) est inférieure à ce seuil (S1), i.e. R1<S1.

Bien entendu, en fonction de l'opération, c'est-à-dire du traitement logique, effectuée pour déterminer l'information de régularité de massage (R1), la logique précédente peut être inversée, c'est-à-dire que le massage peut être considéré comme étant réalisé manuellement lorsque l'information représentative (R1) est inférieure à un seuil donné (S1), et réalisé automatiquement lorsque l'information représentative (R1) est supérieure au seuil donné (S1).
- les moyens de traitement de signal sont, en outre, configurés pour déterminer la phase du massage cardiaque, à savoir phase de compression ou phase de relâchement de la cage thoracique, par exemple en déterminant le signe positif (+) ou négatif (-) de la pente de variation du signal de débit et/ou de pression.
- les moyens de traitement de signal sont, en outre, configurés pour déterminer la période (T) ou la fréquence (F) de réalisation du massage cardiaque, et ensuite en déduire une information représentative de la régularité (R2) du massage cardiaque en déterminant la variabilité de la période (T) et/ou de la fréquence de réalisation du massage cardiaque (F), et enfin à comparer cette information de régularité de massage (R2) à un autre seuil mémorisé (S2), pour en déduire, selon le même (ou analogue) traitement logique que précédemment, que le massage cardiaque est automatique ou manuel.
- les moyens de traitement de signal sont, en outre, configurés pour déterminer une information représentative de la régularité du massage cardiaque (σ) en appliquant un opérateur statistique, par exemple un calcul d'écart-type, au signal de pression et/ou de débit, puis à comparer cette information comme précédemment avec une valeur seuil pour en déduire, selon le même (ou analogue) traitement logique que ci-avant, que le massage cardiaque est automatique ou manuel.
- les moyens de traitement de signal sont configurés pour opérer une détection du mode de délivrance d'un massage cardiaque correspondant à une information de délivrance manuelle ou de délivrance automatique du massage cardiaque, et à fournir ladite information aux moyens de sélection de mode ventilatoire de manière à ce que lesdits moyens de sélection de mode ventilatoire opèrent ou commandent un basculement, c'est-à-dire le passage, d'un mode ventilatoire donné à un autre mode ventilatoire donné en fonction du mode de délivrance du massage cardiaque.
- les moyens de mémorisation, notamment de mode ventilatoires, comprennent au moins une mémoire de stockage de données, en particulier une mémoire flash ou analogue.
- il comprend un circuit de gaz apte à véhiculer le gaz respiratoire délivré par la micro-soufflante et destiné à être administré à un patient en arrêt cardiaque.
- la micro-soufflante est pilotée/commandée par des moyens de pilotage.
- la valve expiratoire est pilotée/commandée par des moyens de pilotage.
- la vanne inspiratoire est pilotée/commandée par des moyens de pilotage.
- au moins une partie du circuit de gaz, les moyens de pilotage, les moyens de traitement de signal et les moyens de sélection de mode ventilatoire sont situés dans une carcasse rigide, c'est-à-dire une enveloppe externe formant la coque ou le capotage de l'appareil.
- le circuit de gaz comprend une portion interne agencée dans la carcasse rigide et une portion externe située hors de la carcasse rigide.
- la portion interne du circuit de gaz comprend un conduit de gaz.
- la portion interne du circuit de gaz est en communication fluidique avec une source de gaz de manière à être alimenté avec du gaz délivré par ladite de gaz.
- les moyens de mesure sont agencés :
- soit sur la portion externe du circuit de gaz située hors de la carcasse rigide de manière à opérer des mesures au sein de ladite portion externe,
- soit sur la portion interne du circuit de gaz située dans la carcasse rigide de manière à opérer des mesures au sein de ladite portion interne.
- il comprend plusieurs moyens de mesure, c'est-à-dire des capteurs, dont agencés sur la portion interne du circuit de gaz et sur la portion externe du circuit de gaz.
- il comprend en outre une interface homme-machine (IHM) apte à, c'est-à-dire conçue pour, afficher des informations incluant au moins une information relative à la réalisation d'un massage cardiaque sur le patient en arrêt cardiaque.
- l'interface homme-machine comprend un écran d'affichage et/ou de visualisation d'informations, tel un écran digital, en particulier un écran tactile, des témoins lumineux, en particulier LEDs.
- il comprend en outre un ou des moyens de génération de signal d'alerte aptes à, c'est-à-dire adaptés ou conçus pour, émettre une information et/ou un signal sonore, relatif au début ou à l'arrêt d'un massage cardiaque ou une information de type alarme indiquant que le massage cardiaque délivré est hors des plages attendues en terme de fréquence, de profondeur,...
- les moyens de génération de signal d'alerte comprennent un haut-parleur pour émettre un signal sonore.
- les moyens de génération de signal d'alerte sont pilotés par les moyens de pilotage
- la portion externe du circuit de gaz est en communication fluidique avec une interface respiratoire, en particulier un masque respiratoire ou une sonde d'intubation.
- la portion externe du circuit de gaz comprend un tube ou conduit flexible.
- la source de gaz comprend une source d'air (env. 21 vol. % d'O₂) ou d'air enrichi en oxygène (>21 vol.% d'O₂ environ)
- la carcasse comprend au moins une poignée de portage facilitant le transport de l'appareil par un utilisateur.
- la carcasse comprend au moins un dispositif d'accrochage permettant l'accroche de l'appareil de ventilation à un support, par exemple une tringle au sein d'un véhicule d'urgence ou un barreau de lit ou de brancard.
- il comprend des moyens d'alimentation en courant électrique, par exemple une (ou plusieurs) batterie ou analogue, rechargeable ou non, ou un (ou plusieurs) câble et une (ou plusieurs) prise de raccordement au secteur électrique.
- il comporte en outre des moyens de réglage et de sélection, tel que bouton-poussoir, touche d'activation, curseur ou analogue, permettant au personnel soignant d'intervenir au niveau du ventilateur, par exemple d'indiquer au ventilateur, la réalisation d'un massage cardiaque, de lui confirmer une détection d'un massage cardiaque, de lui confirmer une détection d'un massage cardiaque délivré manuellement ou automatiquement, de lui indiquer le type d'interface respiratoire utilisée (masque, sonde d'intubation...), de modifier un ou des paramètres de ventilation mécanique proposés automatiquement par le ventilateur, ou tout autre indication.
- il comprend des moyens de pilotage incluant les moyens de traitement de signal.
- il comprend une micro-soufflante en communication fluidique avec la branche inspiratoire du circuit de gaz de manière à l'alimenter en gaz délivré par ladite micro-soufflante et les moyens de pilotage sont configurés pour commander ladite micro-soufflante de manière à ajuster la pression ou la fréquence du gaz délivré, typiquement de l'air éventuellement enrichi en oxygène.
- selon un autre mode de réalisation, il comprend une première vanne pilotée agencée sur ledit circuit de gaz, en particulier sur la branche inspiratoire dudit circuit, permettant de réguler la circulation de gaz alimentant ledit circuit, ledit gaz provenant d'une canalisation extérieure raccordée à l'appareil ou d'une micro-soufflante, et les moyens de pilotage sont configurés pour commander ladite première vanne pilotée de manière à ajuster la pression ou la fréquence du gaz délivré, typiquement de l'air éventuellement enrichi en oxygène.
- les moyens de pilotage opèrent une modification d'un ou des paramètres de la ventilation en cours de délivrance, en fonction du mode de délivrance du massage cardiaque déterminé par les moyens de traitement de signal. Le ou les paramètres modifiés peuvent être la pression basse de ventilation, la pression haute de ventilation, le volume délivré, la fréquence de ventilation, la fraction d'oxygène délivrée et tout autre paramètre définissant la ventilation délivrée.
- l'écran d'affichage et/ou de visualisation d'informations est configuré pour opérer un affichage du mode ventilatoire sélectionnée et/ou du mode de délivrance du massage cardiaque.
- le flux de gaz est délivré par une micro-soufflante équipant l'appareil d'assistance respiratoire ou provient d'une canalisation d'alimentation en gaz raccordée à l'appareil d'assistance respiratoire, en particulier une canalisation d'alimentation en gaz alimentant une prise murale, elle-même connectée fluidiquement à l'appareil d'assistance respiratoire mettant en œuvre le procédé de l'invention.
- le circuit de gaz comprend une branche inspiratoire apte à véhiculer un gaz respiratoire destiné à être administré à un patient en arrêt cardiaque pendant une réanimation cardio-pulmonaire.
- la branche inspiratoire comprend une vanne inspiratoire et un capteur de débit inspiratoire
- le circuit de gaz comprend en outre une branche expiratoire en communication fluidique avec l'atmosphère via un orifice de sortie de gaz, et comportant une valve expiratoire et un capteur de débit expiratoire.
- les moyens de pilotage commandent la micro-soufflante motorisée et/ou la vanne inspiratoire et la valve expiratoire de manière à délivrer du gaz alternativement :
   i) entre une première pression basse (PB1) donnée et une première pression haute (PH1) donnée, avec PH1>PB1, en cas de détection d'un massage cardiaque délivré manuellement et
   ii) entre une deuxième pression basse (PB2) donnée et une deuxième pression haute (PH2) donnée, avec PH2>PB2, en cas de détection d'un massage cardiaque délivré automatiquement, et avec PB2>PB1 et/ou PH2>PH1.
- à titre indicatif, les niveaux de première et deuxième pressions basses (PB1, PB2) sont typiquement compris entre environ 3 et 10 cm d'eau, alors que les niveaux de première et deuxième pressions hautes (PH1, PH2) sont typiquement compris entre environ 8 et 50 cm d'eau.
- de préférence, en présence d'un massage cardiaque manuel, la première pression basse (PB1) est de l'ordre de 5 cm d'eau et la première pression haute (PH1) est de l'ordre de 15 cm d'eau, alors qu'en présence d'un massage cardiaque automatique, la deuxième pression basse (PB2) est de l'ordre de 8 cm d'eau et la deuxième pression haute (PH2) est de l'ordre de 18 cm d'eau.
- les moyens de pilotage commandant la micro-soufflante comprennent une carte électronique, par exemple à microcontrôleur et algorithme(s).

La présente invention va maintenant être décrite plus en détail en références aux Figures annexées parmi lesquelles:
- les Figures 1A et 1B représentent deux modes de réalisation d'un appareil d'assistance respiratoire selon la présente invention;
- les Figures 2A et 2B schématisent les variations provoquées par un massage cardiaque manuel ou automatique, sur un signal de pression mesuré par un appareil d'assistance respiratoire selon l'invention; et
- la Figure 3 schématise les adaptations ventilatoires réalisées par un appareil d'assistance respiratoire selon la présente invention en réponse à la détection du mode de délivrance d'un massage cardiaque appliqué à un patient.

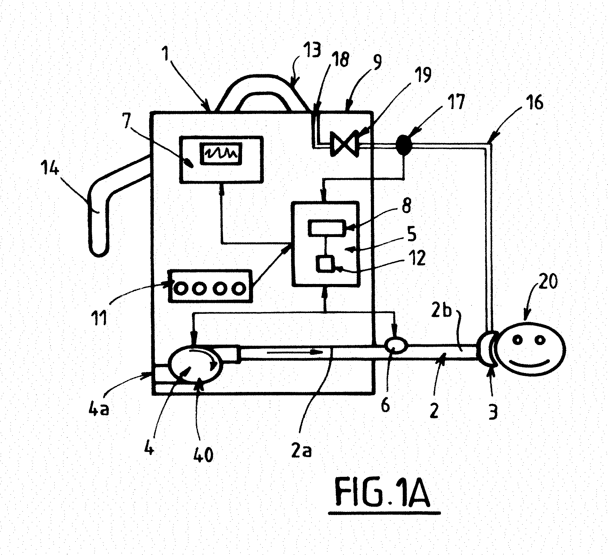

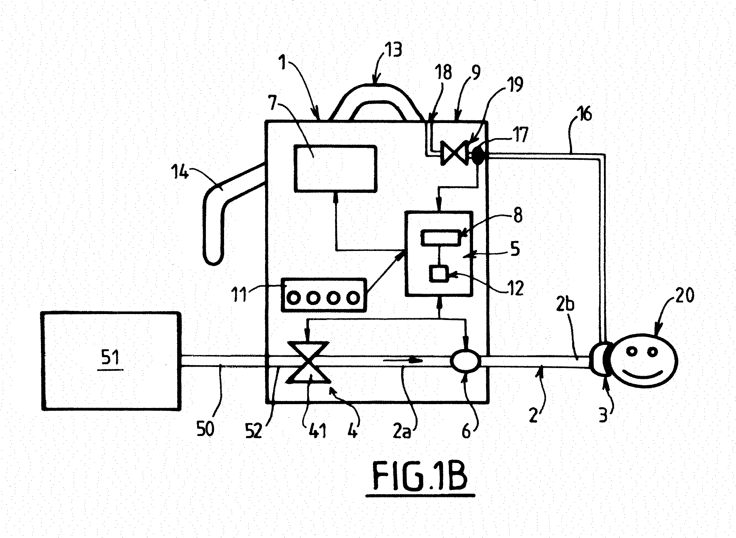

Les Figures 1A et 1B schématisent deux modes de réalisation d'un appareil d'assistance ventilatoire ou ventilateur médical 1 selon la présente invention.

Le ventilateur 1 des Figures 1A et 1B comprend des moyens d'alimentation en gaz 4, i.e. une source de gaz, qui est :
- une micro-soufflante motorisée 40 dans le mode de réalisation de la Figure 1A, encore appelée turbine, délivrant un flux de gaz d'assistance respiratoire, typiquement un flux d'air ou d'air enrichi en oxygène, ou
- une vanne inspiratoire pilotée 41 alimentée en gaz, via un conduit interne 52, lui-même en communication fluidique avec un réservoir de gaz ou une prise murale 51 de fourniture de gaz reliée à un réseau de canalisations de gaz, par l'intermédiaire d'un conduit souple 50 raccordant le réservoir de gaz ou la prise murale 51 au conduit interne 52, dans le mode de réalisation de la Figure 1B.

Dans tous les cas, un circuit ventilatoire 2, 16, encore appelé circuit patient, comprenant un ou plusieurs passages, conduits ou lignes de gaz, permet de relier fluidiquement les moyens d'alimentation en gaz 4 du ventilateur 1 aux voies aériennes d'un patient 20, par l'intermédiaire d'une interface patient 3, par exemple un masque respiratoire ou une sonde d'intubation.

Le circuit ventilatoire 2, 16 comprend au moins une branche inspiratoire 2 permettant de convoyer le gaz respiratoire au patient 20.

Il peut comporter également une branche expiratoire 16 conçue pour recueillir les gaz expirés par le patient 20 qui sont riches en CO₂, comme illustré sur les Figures 1A et 1B. La branche expiratoire 16 comprend un capteur de débit expiratoire 17, par exemple un capteur à fil chaud, relié électriquement à des moyens de pilotage 5 comprenant ici des moyens de traitement de signal 8, ainsi qu'une valve expiratoire 19 pilotée par les moyens de pilotage 5. La branche expiratoire 16 communique, à son extrémité aval, avec l'atmosphère via un orifice de sortie de gaz 18, alors que son extrémité amont est reliée soit à la branche inspiratoire 2, via une pièce en Y, soit directement à l'interface patient 3.

Des moyens de mesure 6 sont prévus, tel un (ou plusieurs) capteur de pression ou de débit, lesquels sont aptes à et conçus pour mesurer au moins un paramètre représentatif du flux de gaz choisi parmi la pression du gaz, le débit de gaz insufflé par le respirateur, le débit de gaz expiré par le patient 20 et la vitesse de rotation de la micro-soufflante 40, et à délivrer au moins un signal représentatif dudit au moins un paramètre mesuré, à savoir un signal de pression ou un signal de débit.

Avantageusement, les moyens de mesure 6 comprennent un capteur de pression dont la prise de pression est agencée de manière à permettre une mesure de la pression gazeuse régnant dans la branche inspiratoire 2 du circuit ventilatoire. Dans ce cas, le paramètre représentatif du flux de gaz est donc la pression du gaz dans la branche inspiratoire 2 du circuit de gaz ventilatoire.

Dans le mode de réalisation illustré sur des Figures 1A et 1B, la prise de pression faisant office de moyens de mesure 6, est agencée hors du ventilateur. Toutefois, elle peut également se trouver dans le ventilateur 1, selon un autre mode de réalisation.

Une fois la mesure du (ou des) paramètre(s) représentatif(s) du flux de gaz opérée, typiquement une (ou des) valeur de pression, ce paramètre mesuré est converti en au moins un signal représentatif du flux de gaz, qui est alors transmis à et analysé par des moyens de traitement de signal 8 pour en déduire au moins une information relative au mode de délivrance d'un massage cardiaque opéré sur le patient en arrêt cardiaque, à savoir un massage manuel ou automatique par une planche à masser par exemple.

Comme déjà dit, les moyens de traitement de signal 8 font partie des moyens de pilotage 5 du ventilateur 1 et comprennent une ou plusieurs cartes électroniques.

La transmission du (ou des) signal est transmis par les moyens de mesure 6 aux moyens de traitement de signal 8 via une connectique adaptée, c'est-à-dire des liaisons électriques, tel que câbles ou autres.

Lorsqu'ils les reçoivent, les moyens de traitement de signal 8 en déduisent ou déterminent selon la présente invention, après détection automatique ou indication par l'utilisateur qu'un massage cardiaque est en cours de réalisation sur le patient 20, que le massage en cours de réalisation est délivré manuellement ou automatiquement.

Les moyens de traitement de signal 8 comprennent de préférence un microprocesseur programmé avec un (ou plusieurs) algorithme(s) de traitement.

En fonction de l'information de massage cardiaque déduite, des moyens de sélection de mode ventilatoire du ventilateur 1 opèrent une recherche et une sélection automatique d'un mode ventilatoire donné parmi plusieurs modes ventilatoires mémorisés dans des moyens de mémorisation 12, et commandent ensuite, via les moyens de pilotage 5, l'appareil d'assistance respiratoire 1 en appliquant le mode ventilatoire ainsi sélectionné, en particulier un mode pour massage manuel (premier mode) et un mode pour massage automatique (second mode).

Comme déjà dit, dans le cadre de l'invention, on appelle « mode ventilatoire », un ensemble de paramètres ventilatoires, tel que pression, débit, volume de gaz délivré... , utilisés pour configurer l'appareil de ventilation et déterminer ainsi les caractéristiques de la ventilation délivrée au patient. A ces paramètres ventilatoires peuvent s'ajouter des monitorages des échanges gazeux entre le patient et l'appareil de ventilation, tel que le volume inspiré, le volume expiré, pressions, concentration en CO₂, concentration en oxygène...

Les différents modes ventilatoires sont mémorisés dans des moyens de mémorisation 12 qui comprennent au moins une mémoire de stockage de données, en particulier une mémoire flash ou analogue.

Les moyens de sélection de mode ventilatoire sont donc aptes à et conçus pour, i.e. configurer pour, retrouver et sélectionner au sein des moyens de mémorisation 12, un mode ventilatoire donné mémorisé, en fonction de l'information représentative d'un mode de massage cardiaque manuel ou automatique fournie par les moyens de traitement de signal 8.

Typiquement, on mémorise dans les moyens de mémorisation 12, plusieurs modes ventilatoires différents comprenant un premier mode ventilatoire ou mode pour massage manuel, et un second mode ventilatoire ou mode pour massage automatique.

Le ventilateur médical 1 de l'invention comporte donc des moyens de traitement 8 aptes à et conçus pour adapter automatiquement, c'est-à-dire sans intervention humaine, les paramètres de la ventilation mécanique délivrée au patient 20, en fonction du mode de délivrance du massage cardiaque détecté par les moyens de traitement de signal 8, dans le but d'assurer une ventilation optimale du patient 20.

L'adaptation peut se faire par modification d'un ensemble de paramètres de la ventilation sélectionnée par l'utilisateur, tels que pressions, volume, fréquence, concentration en oxygène de la ventilation délivrée par le ventilateur... Les valeurs prises par ces paramètres en cas de détection d'un massage manuel et en cas de détection d'un massage automatique, par les moyens de traitement de signal 8, sont mémorisés dans des moyens de mémorisation 12.

L'adaptation peut également se faire par modification d'un mode ventilatoire sélectionné par l'utilisateur, en cas de détection par les moyens de traitement de signal 8 d'un massage cardiaque manuel ou automatique. Dans ce cas, il s'opère un basculement du mode ventilatoire sélectionné vers le mode ventilatoire dédié au massage cardiaque détecté, qu'il soit manuel ou automatique, et mémorisé dans des moyens de mémorisation 12, telle une mémoire.

De préférence, on mémorise au moins :
- un premier mode ventilatoire ou « mode pour massage manuel » comprenant une première valeur de pression basse PB1 et/ou une première valeur de pression haute PH1, avec PH1>PB1, données, lequel premier mode est mise en œuvre en cas de détection d'un massage cardiaque délivré manuellement ; et
- un second mode ventilatoire ou « mode pour massage automatique » comprenant une seconde valeur de pression basse PB2 et/ou une deuxième valeur de pression haute PH2, avec PH2>PB2, PH2≥PH1 et PB2>PB1, données, lequel second mode est mise en œuvre en cas de détection d'un massage cardiaque délivré automatiquement.

Lorsque les moyens de traitement 8 déterminent que le massage cardiaque est délivré automatiquement, il s'opère alors un basculement automatique du premier mode ventilatoire vers le second mode ventilatoire de manière à modifier les valeurs de pression basse et/ou de pression haute par mise en œuvre du second mode ventilatoire en lieu et place du premier mode ventilatoire.

Ainsi, selon un mode de ventilation dit « barométrique », on peut opérer une régulation alternée de pression entre plusieurs niveaux de pression comprenant des niveaux de pression basse PB1, PB2 et des niveaux de pression haute PH1, PH2, avec PH2≥PH1 et PB2>PB1, les pressions PB1 et PH1 étant mises en œuvre en cas de détection d'un massage cardiaque délivré manuellement, et les pressions PB2 et PH2 étant mises en œuvre en cas de détection d'un massage cardiaque délivré automatiquement.

Par exemple, on peut fixer et enregistrer des valeurs de première pression basse PB1 de l'ordre de 5 cm d'H₂O, de première pression haute (PH1) de l'ordre de 15 cm d'H₂O, de deuxième pression basse PB2 de l'ordre de 10 cm d'H₂O et de deuxième pression haute PH2 de l'ordre de 20 cm d'H₂O.

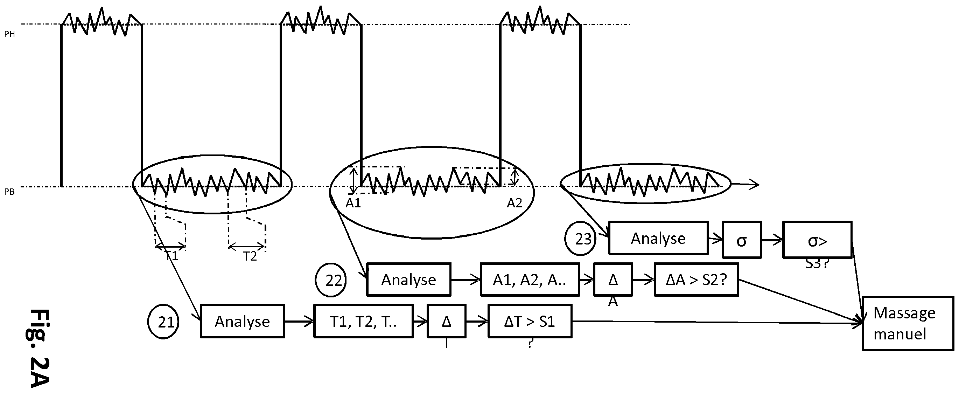

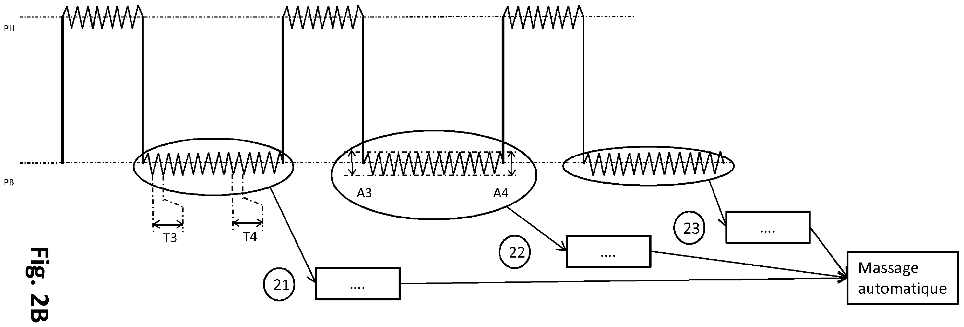

Les paramètres adaptés peuvent être dans le cadre d'une ventilation barométrique entre plusieurs niveaux de pression, les pressions délivrées, en particulier la pression régulée pendant le niveau bas de ventilation, appelée pression basse PB, et la ou les pressions hautes PH1, PH2... régulée(s) pendant le niveau haut, ou encore la fréquence F de ventilation, la durée de maintien de la pression basse, la durée de maintien de la pression haute, la pente de montée en pression, le volume délivré à l'insufflation, la nature du gaz délivré...L'adaptation des niveaux de pression est illustrée sur la Figure 2.

Par ailleurs, le ventilateur 1 peut aussi comporter des moyens de changement des algorithmes d'alarmes, par exemple d'alarmes visant à surveiller le rythme respiratoire du patient, la pression délivrée par le respirateur ou le volume délivré par le respirateur, aptes à et conçus pour passer d'un premier algorithme a un second algorithme, en réponse a une détection par les moyens de traitement de signal, de la réalisation d'un massage cardiaque manuel ou automatique sur le patient, afin de ne pas alarmer à tort et donc d'éviter de perturber inutilement le secouriste ou analogue, en train de secourir le patient 20.

Le ventilateur 1 et ses composants requérant de l'énergie pour fonctionner sont alimentés, directement ou indirectement, en courant électrique issu d'une ou plusieurs batteries, rechargeables ou non, de l'alimentation électrique du véhicule de secours qu'il équipe ou du secteur électrique, donc à une tension typiquement comprise entre 12 et 230V. Si besoin est, il peut intégrer un convertisseur de courant conçu pour abaisser la tension d'alimentation à une tension d'utilisation de valeur inférieure.

Enfin, une interface homme-machine ou IHM 7, tel un écran d'affichage et de visualisation, tel un écran tactile, permet d'afficher et donc à utilisateur de visualiser des informations relatives à la ventilation délivrée, tel que la pression et le débit mesurés par les moyens de mesure 6.

Sont également prévus, des moyens de réglage et de sélection 11, par exemple des boutons poussoirs ou rotatifs, des curseurs, des touches d'activation ou de sélection, ou analogue permettant au personnel soignant d'indiquer au ventilateur 1, la réalisation d'un massage cardiaque et/ou de confirmer au ventilateur 1, la détection qui a été faite du mode de délivrance du massage cardiaque, d'indiquer au ventilateur le type d'interface avec le patient, par exemple masque, sonde d'intubation...

Ces moyens de réglage et de sélection 11 permettent également de modifier, si besoin est, les paramètres de la ventilation mécanique proposés automatiquement par le ventilateur 1, voire même, selon le mode de réalisation considéré, de pouvoir indiquer au ventilateur 1 un changement de la nature du gaz mis en œuvre, par exemple le passage d'air à un mélange air/oxygène ou un changement de teneur en oxygène d'un mélange air/oxygène.

Comme on le voit sur la Figure 1, au moins une partie du circuit de gaz 2, 16, les moyens de traitement de signal 8 et la source de gaz 4 sont agencés dans un capotage ou une carcasse rigide 9 qui forme l'enveloppe externe de l'appareil 1. Cette carcasse 9 inclut ou porte en outre d'autres composants, tel que l'IHM 7, la (ou les) mémoire 12, les moyens de réglage et sélection 11...

La branche inspiratoire 2 du circuit de gaz comprend deux portions distinctes, à savoir une portion interne 2a agencée dans la carcasse rigide 9, par exemple un conduit de gaz, et une portion externe 2b située hors de la carcasse rigide 9 incluant par exemple un tuyau flexible. La portion interne 2a de la branche inspiratoire 2 est en communication fluidique avec la micro-soufflante motorisée 40 de la Figure 1A ayant une prise ou entrée d'air 4a communiquant avec l'atmosphère ambiante, ou selon le cas, avec la vanne inspiratoire 41 pilotée de la Figure 1B, de manière à alimenter ladite portion interne 2a avec de l'air, éventuellement enrichi en oxygène.

La micro-soufflante motorisée 40 (Fig. 1A) ou la vanne inspiratoire 41 pilotée (Fig. 1B) est pilotée par les moyens de pilotage 5 qui comprennent, de préférence, une carte électronique à microprocesseur, tel un microcontrôleur, mettant en œuvre un (ou des) algorithme(s).

De préférence, les moyens de pilotage 5 incorporent aussi les moyens de traitement de signal 8, et sont configurés pour piloter la micro-soufflante motorisée 40 ou la vanne inspiratoire 41 pilotée en fonction des signaux transmis par les moyens de traitement de signal 8.

Par ailleurs, la portion externe 2b de la branche inspiratoire 2 du circuit de gaz 2, 16 située hors de la carcasse rigide 9 est, quant à elle, en communication fluidique avec, du côté amont, la portion interne 2a de la branche inspiratoire 2 et, du côté aval, avec l'interface respiratoire 3, tel un masque ou une sonde d'intubation, de manière à assurer une continuité fluidique entre les moyens d'alimentation en gaz 4 et le patient 20, et permettre d'acheminer le gaz respiratoire jusqu'aux voies aériennes dudit patient.

Sur les Figures 1A et 1B, les moyens de mesure 6, typiquement un (ou plusieurs) capteur, sont agencés sur la portion externe 2b de la branche inspiratoire 2 située hors de la carcasse rigide 9 de manière à opérer les mesures désirées, par exemple de pression et/ou de débit, au sein de ladite portion externe 2b. Bien entendu, les moyens de mesure 6 peuvent être aussi agencés à l'intérieur de la carcasse 9. Dans tous les cas, la liaison entre les moyens de mesure 6 et les moyens de traitement 8 et/ou les moyens de pilotage 5, et donc le transfert des signaux de mesure, se fait au moyen de liaisons, filaires par exemple.

Optionnellement, la carcasse 9 peut également comprendre au moins une poignée de portage 13 pour faciliter le transport de l'appareil 1 par l'utilisateur, ce qui est indispensable dans certaines situations d'urgence, et/ou un dispositif d'accrochage 14 permettant l'accroche de l'appareil 1 de ventilation à un support, par exemple une tringle au sein d'un véhicule d'urgence ou un barreau de lit ou de brancard.

Les Figures 2A et 2B représentent les variations provoquées par un massage cardiaque, sur un signal de pression (P) mesuré au fil du temps par un appareil d'assistance respiratoire 1 selon la présente invention, par exemple celui de la Figure 1A. Pour des raisons pratiques, seul le signal de pression (P) est représenté, le signal de débit, non représenté, présente lui aussi des variations provoquées par le massage cardiaque. Par analogie les opérations appliquées au signal de pression, selon la présente invention, peuvent être appliquées au signal de débit.

Dans les conditions de ventilation de la Figure 2A, la ventilation en pression contrôlée se déroule entre des premières pressions basse PB et haute PH, et un massage cardiaque délivré manuellement est effectué sur le patient 20 auquel du gaz est fourni par le ventilateur 1.

Les phases de compression et de décompression du massage cardiaque provoquent des variations de pression P. La pression est mesurée, de préférence en continu, par les moyens de mesure 6 de pression, tel un capteur de pression, et transmise pour analyse aux moyens de traitement de signal 8, comme expliqué ci-avant.

Les phases de compression et de décompression du massage cardiaque provoquent des variations de débit Q. Le débit est mesuré, de préférence en continu, par des moyens de mesure de débit 6, 17, tel des capteurs de débit, et transmis pour analyse aux moyens de traitement de signal 8.

Lors d'un massage manuel, la fréquence des compressions thoraciques n'est pas constante et elle varie autour de la fréquence recommandée, à savoir typiquement autour de 100 compressions/min. Il en résulte que le temps écoulé T1, T2, Tx... entre deux compressions, c'est-à-dire le temps qui s'écoule entre deux décompressions, n'est pas constant, comme visible sur la Figure 2A.

L'amplitude A des compressions délivrées pendant le massage cardiaque n'est, elle non plus, pas constante.

De là, les variations de pression P et de débit Q qui en résultent A1, A2, Ax..., ne sont pas non plus constantes.

De façon générale, l'ensemble des paramètres calculés à partir des signaux de pression P et de débit Q n'ont pas de caractéristiques constantes.

A l'inverse, comme illustré en Figure 2B, lors d'un massage cardiaque réalisé par un dispositif automatique, telle une planche à masser, la fréquence F des compressions thoraciques ainsi que leurs amplitudes A sont constantes. Il en résulte alors que les caractéristiques, telles que période, fréquence et amplitude, des signaux de pression et de débit mesurés sont également constantes.

Les moyens de traitement 8 de l'appareil respiratoire1 exploite ces différences mesurables par exploitation des signaux de pression P et/ou de débit Q pour déterminer le mode de réalisation du massage cardiaque, à savoir manuel ou automatique, et adapter en réponse à cette détermination, les paramètres de la ventilation fournie au patient 20 en allant rechercher dans les moyens de mémorisation 12, le mode ventilatoire le plus adapté au massage cardiaque détecté, i.e. manuel ou automatique.

En fait, les moyens de traitement de signal 8 déterminent (en 21 sur Fig. 2A) la période T1, T2, ... ou la fréquence F de réalisation du massage cardiaque, pendant la ventilation. A partir de ces informations, les moyens de traitement de signal 8 déterminent la variation ΔT de la période ou de la fréquence au cours du temps.

Cette variation ΔT est comparée à un seuil S1. Le seuil S1 est choisi de tel sorte que lorsque la variation ΔT est supérieure au seuil S1 (i.e. ΔT>S1), le massage cardiaque est considéré comme délivré manuellement, alors qu'à l'inverse, lorsque la variation ΔT est inférieure au seuil S1 (i.e. ΔT<S1), le massage cardiaque est considéré comme délivré automatiquement. Bien entendu, en fonction du mode de calcul utilisé pour obtenir la variation ΔT, la logique peut être inversée.

Ensuite (en 22), les moyens de traitement de signal 8 déterminent l'amplitude A1, A2, ... des variations de pression et/ou de débit engendrées par le massage cardiaque. A partir de ces informations, les moyens de traitement de signal 8 déterminent la variation ΔA de l'amplitude des variations A1, A2,... au fil du temps. Comme précédemment, la variation ΔA est comparée à un seuil S2 choisi de sorte que, lorsque la variation ΔA est supérieure au seuil S2 (i.e. ΔA>S2), le massage cardiaque est considéré comme délivré manuellement, alors que lorsque la variation ΔA est inférieure au seuil S2 (i.e. ΔA<S2), le massage cardiaque est considéré comme délivré automatiquement. Là encore, en fonction du mode de calcul utilisé pour obtenir la variation ΔA, la logique peut être inversée.

Les moyens de traitement de signal 8 appliquent alors (en 23) un opérateur statistique au signal de pression P et/ou de débit Q dans le but de déterminer une information σ, image de la dispersion du signal considéré, qui est comparée à un seuil S3. Le seuil S3 est choisi de manière à ce que, lorsque quand l'information σ est supérieure au seuil S3 (i.e. ΔA>S3), le massage cardiaque est considéré comme délivré manuellement, alors que lorsque l'information σ est inférieure au seuil S3 (i.e. ΔA<S3), le massage cardiaque est considéré comme délivré automatiquement. Là encore, en fonction du mode de calcul utilisé pour obtenir l'information σ, la logique peut être inversée.

Le massage est considéré, par les moyens de traitement 8 de l'appareil d'assistance respiratoire 1, comme étant délivré manuellement, lorsque l'un ou plusieurs des traitements opérés précédemment (en 21, 22 et 23) déterminent à partir des signaux de pression et/ou de débit que le massage est délivré manuellement.

Les Figures 2A et 2B permettent de comparer les traitements opérés (en 21, 22, 23) pour une mesure effectuée dans des conditions de massage manuel (Fig. 2A) et pour une mesure effectuée dans des conditions de massage automatique (Fig. 2B), à savoir ici au moyen d'une planche à masser.

Comme on le voit, la régularité du massage automatique entraine que les périodes mesurées T3, T4... sont égales ou très similaires les unes des autres. Dans ces conditions, la détermination d'une variabilité ΔT (en 21) conduit à une valeur ΔT nulle ou proche de 0. La variabilité ΔT est alors inférieure au seuil S1, ce qui est représentatif d'un massage cardiaque délivré automatiquement, comme expliqué ci-avant.

De la même façon, la régularité du massage cardiaque automatique implique également que l'amplitude A3, A4... des variations de pression et/ou de débit engendrées par le massage cardiaque est constante ou n'évolue que faiblement d'un cycle de compression/décompression à un autre, et de ce fait une détermination (en 22) d'une variabilité ΔA nulle ou proche de 0, donc là encore, inférieure au seuil S1, confirmant ainsi un massage cardiaque délivré automatiquement.

Il en va de même de la détermination (en 23) de l'information σ, i.e. l'image de la dispersion du signal considéré, et de sa comparaison au seuil S3, qui conduisent à conclure aussi à un massage cardiaque délivré automatiquement.

Le massage est considéré par les moyens de traitement 8 de l'appareil d'assistance respiratoire 1, comme étant délivré automatiquement, lorsque l'un ou plusieurs des paramètres (obtenus en 21, 22, 23) obtenus à partir des signaux de pression et/ou de débit, correspondent à un massage régulier, donc délivré automatiquement.

Sur les Figures 2A et 2B, les étapes de traitement 21, 22, 23 sont appliquées aux signaux mesurés pendant la phase expiratoire de la ventilation et à une ventilation entre deux niveaux de pression PB, PH. Toutefois, elles peuvent être appliquées aux signaux mesurés pendant n'importe quelle phase de la ventilation et/ou à tout mode de ventilation, notamment les ventilations à volume contrôlé, à pression continue...

Le traitement opéré (en 21, 22, 23) peut être appliqué simultanément aux différents signaux mesurés ou, selon le cas, successivement.

De façon analogue, pour effectuer la détection du type de massage, on peut utiliser l'un ou plusieurs des paramètres susmentionnés, c'est-à-dire mettre en œuvre une ou plusieurs des étapes de traitement 21, 22, 23, et ce, sur un ou plusieurs cycles de compression/décompression, c'est-à-dire un seul ou un ensemble de cycles de compression/décompression, ou sur une durée donnée.

Le traitement des mesures (en 21, 22, 23) en continu permet d'obtenir l'information du mode de délivrance du massage cardiaque également en continu. Ainsi, si l'équipe soignante change le mode de délivrance du massage cardiaque pendant la prise en charge, l'appareil d'assistance respiratoire réagit immédiatement en adaptant la ventilation délivrée.

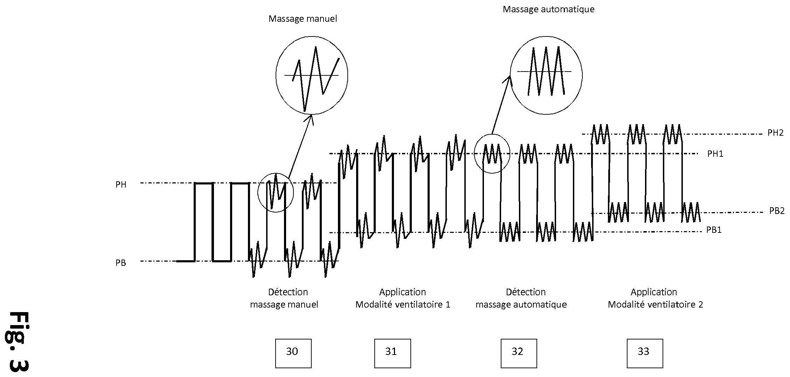

La Figure 3 schématise les adaptations ventilatoires effectuées par un appareil d'assistance respiratoire selon la présente invention, suite à une détection d'un massage manuel ou automatique par les moyens de traitement de signal.

Dans les conditions de ventilation de la Figure 3, la ventilation en pression contrôlée se déroule entre deux niveaux de pression initiale, à savoir une pression basse PB et une pression haute PH, avec PH>PB, et un massage cardiaque délivré manuellement est effectué.

La présence des compressions thoraciques peut être détectée automatiquement par la machine ou signalée par l'utilisateur par activation d'un dispositif de commande.

Les moyens de traitement déterminent (en 30) comme précédemment décrit (21-23 sur Fig. 2A, 2B) que le massage cardiaque est réalisé manuellement. L'utilisateur peut aussi indiquer ou confirmer ce mode de massage manuel au ventilateur par activation d'un dispositif de commande, telle une touche ou analogue.

L'appareil d'assistance respiratoire adapte alors automatiquement (en 31), la ventilation aux conditions du massage manuel, en appliquant le premier mode ventilatoire. La ventilation est alors délivrée entre de nouveaux niveaux de pression basse PB1 (i.e. première pression basse) et haute PH1 (i.e. première pression haute), avec PB1<PH1, PB1>PB et PH1>PH.

Lorsque le mode de réalisation du massage cardiaque change et passe d'un massage manuel à un massage automatique, ce changement est détecté (en 32) par les moyens de traitement de signal, comme expliqué ci-avant. L'utilisateur peut aussi indiquer ou confirmer au ventilateur ce changement de mode par activation d'un dispositif de commande, c'est-à-dire le passage à un massage cardiaque automatique.

L'appareil d'assistance respiratoire adapte alors (en 33) la ventilation aux conditions du massage automatique, en appliquant un second mode ventilatoire. La ventilation est alors délivrée entre les deuxième pressions basse PB2 et haute PH2, avec PB2<PH2, PB2>PB1 et PH2>PH1.

Tout nouveau changement de mode de réalisation du massage cardiaque, par exemple passage du massage automatique à un massage manuel, ou inversement, sera détecté de la même manière par l'appareil ventilatoire et celui-ci adaptera la ventilation aux nouvelles conditions en appliquant un mode ventilatoire adapté au massage détecté, à savoir manuel ou automatique.

Enfin, tout arrêt des compressions thoraciques peut être détecté automatiquement par la machine ou signalé par l'utilisateur par activation d'un dispositif de commande. L'arrêt des compressions thoraciques peut provoquer le passage d'un des modes ventilatoires propre au massage cardiaque à la ventilation en vigueur avant leur application. La ventilation se déroule alors entre les pressions PB et PH

De façon générale, l'invention repose sur une détection du type de massage thoracique effectué, à savoir manuel ou automatique, par analyse par le ventilateur des signaux de pression ou débit qui varient selon le type de massage, et une application automatique d'un mode adapté, par exemple l'application d'un niveau de pression modéré lorsque le massage détecté est manuel, c'est-à-dire pratiqué par un secouriste ou analogue, et d'un niveau de pression supérieur, lorsque le massage détecté est automatique, c'est-à-dire pratiqué par une planche à masser ou analogue.

## Revendications

1. Appareil d'assistance respiratoire (1) comprenant un circuit de gaz (2) conçu pour acheminer un flux de gaz, comprenant :
- des moyens de mesure (6) agencés pour mesurer au moins un paramètre de flux représentatif du flux de gaz acheminé par le circuit de gaz (2) et configurés pour convertir ledit au moins un paramètre de flux en au moins un signal de flux représentatif du flux de gaz,
- des moyens de traitement de signal (8) aptes à et conçus pour traiter ledit au moins un signal de flux fourni par les moyens de mesure (6), et pour en déduire une information représentative d'un mode de massage cardiaque manuel ou d'un mode de massage cardiaque automatique,
- des moyens de mémorisation (12) configurés pour mémoriser plusieurs modes ventilatoires comprenant:
i) un premier mode ventilatoire comprenant des premiers paramètres de ventilation (PB1, PH1) applicables en cas de massage cardiaque manuel, et
ii) un second mode ventilatoire comprenant des seconds paramètres de ventilation (PB2, PH2) applicables en cas de massage cardiaque automatique,
- et des moyens de sélection de mode ventilatoire aptes à et conçus pour retrouver et sélectionner au sein des moyens de mémorisation (12), le premier ou le second mode ventilatoire mémorisé, en fonction de l'information représentative d'un mode de massage cardiaque manuel ou automatique fournie par les moyens de traitement de signal (8).

2. Appareil selon la revendication précédente, **caractérisé en ce que** les moyens de mémorisation (12) sont configurés pour mémoriser :
- un premier mode ventilatoire comprenant des premiers paramètres de ventilation comprenant des premières valeurs de pression (PB1, PH1), et
- un second mode ventilatoire comprenant des seconds paramètres de ventilation comprenant des secondes valeurs de pression (PB2, PH2).

3. Appareil selon l'une des revendications précédentes, **caractérisé en ce que** les moyens de mémorisation (12) sont configurés pour mémoriser :
- un premier mode ventilatoire comprenant des premiers paramètres de ventilation comprenant des valeurs de première pression basse (PB1) et de première pression haute (PH1), avec PH1>PB1, et
- un second mode ventilatoire comprenant des valeurs de deuxième pression basse (PB2) et de deuxième pression haute (PH2), avec PH2≥PH1>PB2 et PB2>PB1.

4. Appareil selon l'une des revendications précédentes, **caractérisé en ce qu'**il comprend :
- des moyens d'alimentation en gaz (4, 40, 41) pour délivrer un flux de gaz dans le circuit de gaz (2) et
- des moyens de pilotage (5) commandant les moyens d'alimentation en gaz () en fonction du mode ventilatoire sélectionné par les moyens de sélection de mode ventilatoire.

5. Appareil selon l'une des revendications précédentes, **caractérisé en ce que** les moyens d'alimentation en gaz (4, 40, 41) comprennent une micro-soufflante motorisée (40) ou un conduit interne (52) équipé d'une vanne inspiratoire (41).

6. Appareil selon l'une des revendications précédentes, **caractérisé en ce que** les moyens de mesure (6) comprennent au moins un capteur de débit ou de pression.

7. Appareil selon l'une des revendications précédentes, **caractérisé en ce que** les moyens de pilotage (5) comprennent les moyens de traitement de signal (8).

8. Appareil selon l'une des revendications précédentes, **caractérisé en ce que** les moyens de pilotage (5) comprennent au moins une carte électronique mettant en œuvre au moins un algorithme.

9. Appareil selon l'une des revendications précédentes, **caractérisé en ce que** les moyens de pilotage commandent la micro-soufflante motorisée et/ou la vanne inspiratoire et la valve expiratoire de manière à délivrer du gaz alternativement :
i) entre une première pression basse (PB1) donnée et une première pression haute (PH1) donnée, avec PH1>PB1, en cas de détection d'un massage cardiaque délivré manuellement et
ii) entre une deuxième pression basse (PB2) donnée et une deuxième pression haute (PH2) donnée, avec PH2>PB2, en cas de détection d'un massage cardiaque délivré automatiquement, et avec PB2>PB1 et/ou PH2≥PH1.

10. Appareil selon l'une des revendications précédentes, **caractérisé en ce que** les première et deuxième pressions basses (PB1, PB2) sont comprises entre 3 et 10 cm d'eau, et les première et deuxième pressions hautes (PH1, PH2) sont comprises entre 8 et 50 cm d'eau.

## Patentansprüche

1. Einrichtung zur Atmungsunterstützung (1), umfassend einen Gaskreislauf (2), der gestaltet ist, um eine Gasströmung zuzuführen, umfassend:
- Messmittel (6), die angeordnet sind, um mindestens einen Strömungsparameter zu messen, der repräsentativ für die durch den Gaskreislauf (2) zugeführte Gasströmung ist und konfiguriert, um den mindestens einen Strömungsparameter in mindestens ein Strömungssignal umzuwandeln, das repräsentativ für die Gasströmung ist,
- Signalverarbeitungsmittel (8), die geeignet und gestaltet sind, um das mindestens eine Strömungssignal, das durch die Messmittel (6) geliefert wird, zu verarbeiten und daraus eine Information abzuleiten, die repräsentativ für einen manuellen Herzmassagemodus oder für einen automatischen Herzmassagemodus ist,
- Speichermittel (12), die konfiguriert sind, um mehrere Beatmungsmodi zu speichern, umfassend:
i) einen ersten Beatmungsmodus, umfassend erste Beatmungsparameter (PB1, PH1), die im Fall einer manuellen Herzmassage anwendbar sind, und
ii) einen zweiten Beatmungsmodus, umfassend zweite Beatmungsparameter (PB2, PH2), die im Fall einer automatischen Herzmassage anwendbar sind,
- und Beatmungsmodus-Auswahlmittel, die geeignet und gestaltet sind, um innerhalb der Speichermittel (12) den ersten oder den zweiten gespeicherten Beatmungsmodus in Abhängigkeit von der für eine manuelle oder automatische Herzmassage repräsentative Information, die durch die Signalbearbeitungsmittel (8) geliefert wird, wiederzufinden und auszuwählen.

2. Einrichtung nach dem vorstehenden Anspruch, **dadurch gekennzeichnet, dass** die Speichermittel (12) konfiguriert sind, um zu speichern:
- einen ersten Beatmungsmodus, umfassend erste Beatmungsparameter, die erste Druckwerte (PB1, PH1) umfassen, und
- einen zweiten Beatmungsmodus, umfassend zweite Beatmungsparameter, die zweite Druckwerte (PB2, PH2) umfassen.

3. Einrichtung nach einem der vorstehenden Ansprüche, **dadurch gekennzeichnet, dass** die Speichermittel (12) konfiguriert sind, um zu speichern:
- einen ersten Beatmungsmodus, umfassend erste Beatmungsparameter, die Werte eines ersten niedrigen Drucks (PB1) und eines ersten hohen Drucks (PH1) umfassen, mit PH1 > PB1, und
- einen zweiten Beatmungsmodus, umfassend Werte eines zweiten niedrigen Drucks (PB2) und eines zweiten hohen Drucks (PH2), mit PH2≥ PH1 > PB2 und PB2 > PB1.

4. Einrichtung nach einem der vorstehenden Ansprüche, **dadurch gekennzeichnet, dass** sie umfasst:
- Gasversorgungsmittel (4, 40, 41), um eine Gasströmung in den Gaskreislauf (2) abzugeben und
- Ansteuerungsmittel (5), die die Gasversorgungsmittel () in Abhängigkeit von dem durch die Beatmungsmodus-Auswahlmittel ausgewählten Beatmungsmodus steuern.

5. Einrichtung nach einem der vorstehenden Ansprüche, **dadurch gekennzeichnet, dass** die Gasversorgungsmittel (4, 40, 41) ein motorisiertes Mikrogebläse (40) oder eine mit einem Einatmungsventil (41) ausgestattete innere Leitung (52) umfassen.

6. Einrichtung nach einem der vorstehenden Ansprüche, **dadurch gekennzeichnet, dass** die Messmittel (6) mindestens einen Durchsatz- oder Drucksensor umfassen.

7. Einrichtung nach einem der vorstehenden Ansprüche, **dadurch gekennzeichnet, dass** die Ansteuerungsmittel (5) die Signalverarbeitungsmittel (8) umfassen.

8. Einrichtung nach einem der vorstehenden Ansprüche, **dadurch gekennzeichnet, dass** die Ansteuerungsmittel (5) mindestens eine elektronische Karte umfassen, die mindestens einen Algorithmus umsetzt.

9. Einrichtung nach einem der vorstehenden Ansprüche, **dadurch gekennzeichnet, dass** die Anteuerungsmittel das motorisierte Mikrogebläse und/oder das Einatmungsventil und das Ausatmungsventil derart steuern, um Gas abwechselnd abzugeben:
i) zwischen einem gegebenen ersten niedrigen Druck (PB1) und einem gegebenen ersten hohen Druck (PH1), mit PH1 > PB1, im Fall einer Detektion einer manuell abgegebenen Herzmassage und
ii) zwischen einem gegebenen zweiten niedrigen Druck (PB2) und einem gegebenen zweiten hohen Druck (PH2), mit PH2 > PB2, im Fall einer Detektion einer automatisch abgegebenen Herzmassage, und mit PB2 > PB1 und/oder PH2≥ PH1.

10. Einrichtung nach einem der vorstehenden Ansprüche, **dadurch gekennzeichnet, dass** der erste und zweite niedrige Druck (PB1, PB2) zwischen 3 und 10 cm Wasser umfasst sind und der erste und zweite hohe Druck (PH1, PH2) zwischen 8 und 50 cm Wasser umfasst sind.

## Claims

1. Respiratory assistance device (1) comprising a gas circuit (2) designed to convey a gas stream, comprising:
- measuring means (6) arranged to measure at least one stream parameter representative of the gas stream conveyed by the gas circuit (2) and configured to convert said at least one stream parameter into at least one stream signal representative of the gas stream,
- signal processing means (8) able to and designed to process said at least one stream signal supplied by the measuring means (6), and to deduce information from it, representative of a manual cardiac massage mode or an automatic cardiac massage mode,
- storage means (12) configured to store several ventilatory modes comprising:
i) a first ventilatory mode comprising first ventilation parameters (PB1, PH1) applicable in case of manual cardiac massage, and
ii) a second ventilatory mode comprising second ventilation parameters (PB2, PH2) applicable in case of automatic cardiac massage,
- and ventilatory mode selection means able to and designed to find and select, within the storage means (12), the first or the second stored ventilatory mode, according to the information representative of a manual or automatic cardiac massage mode supplied by the signal processing means (8).

2. Apparatus according to the preceding claim, **characterised in that** the storage means (12) are configured to store:
- a first ventilatory mode comprising first ventilation parameters comprising first pressure values (PB1, PH1), and
- a second ventilatory mode comprising second ventilation parameters comprising second pressure values (PB2, PH2).

3. Apparatus according to one of the preceding claims, **characterised in that** the storage means (12) are configured to store:
- a first ventilatory mode comprising first ventilation parameters comprising first low-pressure (PB1) and first high-pressure (PH1) values, with PH1 > PB1, and
- a second ventilatory mode comprising second low-pressure (PB2) and second high-pressure (PH2) values, with PH2 ≥ PH1 > PB2 and PB2 > PB1.

4. Apparatus according to one of the preceding claims, **characterised in that** it comprises:
- gas supply means (4, 40, 41) to deliver a gas stream in the gas circuit (2) and
- control means (5) controlling the gas supply means () according to the ventilatory mode selected by the ventilatory mode selection means.

5. Apparatus according to one of the preceding claims, **characterised in that** the gas supply means (4, 40, 41) comprise a motorised micro-fan (40) or an internal conduit (52) equipped with an inspiratory valve (41).

6. Apparatus according to one of the preceding claims, **characterised in that** the measuring means (6) comprise at least one flow or pressure sensor.

7. Apparatus according to one of the preceding claims, **characterised in that** the control means (5) comprise the signal processing means (8).

8. Apparatus according to one of the preceding claims, **characterised in that** the control means (5) comprise at least one electronic board implementing at least one algorithm.

9. Apparatus according to one of the preceding claims, **characterised in that** the control means control the motorised micro-fan and/or the inspiratory valve and the expiratory valve so as to deliver gas alternatively:
i) between a first given low pressure (PB1) and a first given high pressure (PH1), with PH1 > PB1, in case of detecting a cardiac massage delivered manually and
ii) between a second given low pressure (PB2) and a second given high pressure (PH2), with PH2 > PB2, in case of detecting a cardiac massage delivered automatically, and with PB2 > PB1 and/or PH2 ≥ PH1.

10. Apparatus according to one of the preceding claims, **characterised in that** the first and second low pressures (PB1, PB2) are comprised between 3 and 10 cm of water, and the first and second high pressures (PH1, PH2) are comprised between 8 and 50 cm of water.
